(19) Europäisches Patentamt
European Patent Office
Office européen des brevets

(11) **EP 1 353 694 B1**

(12) **EUROPEAN PATENT SPECIFICATION**

(45) Date of publication and mention
of the grant of the patent:
**19.12.2007 Bulletin 2007/51**

(51) Int Cl.:
***A61K 45/06*** (2006.01)  ***A61P 9/00*** (2006.01)
***A61K 31/395*** (2006.01)

(21) Application number: **02704233.2**

(22) Date of filing: **25.01.2002**

(86) International application number:
**PCT/US2002/002013**

(87) International publication number:
**WO 2002/058734 (01.08.2002 Gazette 2002/31)**

(54) **COMBINATIONS OF EZETIMIBE WITH ASPIRINE FOR TREATING VASCULAR CONDITIONS**

KOMBINATIONEN VON EZETIMIBE UND ASPIRIN ZUR BEHANDLUNG VON KARDIOVASKULÄREN ERKRANKUNGEN

COMBINAISONS DU EZETIMIBE ET DE L'ASPIRINE POUR LE TRAITEMENT DES TROUBLES VASCULAIRES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **26.01.2001 US 264396 P**
**26.01.2001 US 264600 P**
**26.01.2001 US 264275 P**
**21.09.2001 US 324123 P**

(43) Date of publication of application:
**22.10.2003 Bulletin 2003/43**

(60) Divisional application:
**07009475.0 / 1 810 693**

(73) Proprietor: **Schering Corporation**
**Kenilworth, NJ 07033-0530 (US)**

(72) Inventors:
• **KOSOGLOU, Teddy**
**Jamison, Bucks County, PA 18929-1178 (US)**
• **RESS, Rudyard, Joseph**
**Flemington, NJ 08822-5910 (US)**
• **STRONY, John**
**Lebanon, NJ 08833 (US)**
• **VELTRI, Enrico, P.**
**Princeton, NJ 08540 (US)**

(74) Representative: **HOFFMANN EITLE**
**Patent- und Rechtsanwälte**
**Arabellastrasse 4**
**81925 München (DE)**

(56) References cited:
**EP-A- 1 036 563**  **WO-A-01/96347**
**WO-A-02/26729**  **WO-A-02/50027**
**WO-A-02/50060**  **WO-A-02/50068**
**WO-A-95/35277**  **WO-A-02/064094**

• **KOSOGLOU T. ET AL: "Coadministration of ezetimibe and fenofibrate leads to favorable effects on Apo CII and LDL subfractions" ATHEROSCLEROSIS, vol. 2, no. Suppl, 2001, page 89 XP001132089**
• **DAVIS H. R. ET AL: "The synergistic hypocholesterolemic activity of the potent cholesterol absorption inhibitor, ezetimibe, in combination with 3-hydroxy-3-methylglutaryl coenzyme A reductase inhibitors in dogs" METABOLISM, vol. 50, no. 10, October 2001 (2001-10), pages 1234-1241, XP008011711**

EP 1 353 694 B1

**Description**

FIELD OF THE INVENTION

The present invention relates to compositions and therapeutic use for treating vascular conditions in mammals comprising the blood modifier aspirin and the sterol absorption inhibitor ezetimibe.

BACKGROUND OF THE INVENTION

Vascular disease is a term that broadly encompasses all disorders of blood vessels including small and large arteries and veins and blood flow. The most prevalent form of vascular disease is arteriosclerosis, a condition associated with the thickening and hardening of the arterial wall. Arteriosclerosis of the large vessels is referred to as atherosclerosis. Atherosclerosis is the predominant underlying factor in vascular disorders such as coronary artery disease, aortic aneurysm, arterial disease of the lower extremities and cerebrovascular disease.

One major risk factor for arteriosclerosis is high serum cholesterol. A total cholesterol level in excess of 225-250 mg/dl is associated with significant elevation of risk of vascular disease, particularly coronary heart disease.

Cholesteryl esters are a major component of atherosclerotic lesions and the major storage form of cholesterol in arterial wall cells. Formation of cholesteryl esters is also a step in the intestinal absorption of dietary cholesterol. Thus, inhibition of cholesteryl ester formation and reduction of serum cholesterol can inhibit the progression of atherosclerotic lesion formation, decrease the accumulation of cholesteryl esters in the arterial wall, and block the intestinal absorption of dietary cholesterol.

The regulation of whole-body cholesterol homeostasis in mammals and animals involves the regulation of dietary cholesterol and modulation of cholesterol biosynthesis, bile acid biosynthesis and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis. When intestinal cholesterol absorption is reduced, by whatever means, less cholesterol is delivered to the liver. The consequence of this action is decreased hepatic lipoprotein (VLDL) production and an increase in the hepatic clearance of plasma cholesterol, mostly as LDL. Thus, the net effect of inhibiting intestinal cholesterol absorption is a decrease in plasma cholesterol levels.

U.S. Patents Nos. 5,767,115, 5,624,920, 5,668,990, 5,656,624 and 5,688,787, respectively, disclose hydroxy-substituted azetidinone compounds and substituted β-lactam compounds useful for lowering cholesterol and/or in inhibiting the formation of cholesterol-containing lesions in mammalian arterial walls. U.S. Patents Nos. 5,846,966 and 5,661,145, respectively, disclose hydroxy-substituted azetidinone compounds or substituted β-lactam compounds in combination with HMG CoA reductase inhibitors for preventing or treating atherosclerosis and reducing plasma cholesterol levels.

PCT Patent Application No. WO 00/38725 discloses cardiovascular therapeutic combinations including an ileal bile acid transport inhibitor or cholesteryl ester transport protein inhibitor in combination with a fibric acid derivative, nicotinic acid derivative, microsomal triglyceride transfer protein inhibitor, cholesterol absorption antagonist, phytosterol, stanol, antihypertensive agent or bile acid sequestrant.

U.S. Patent No. 5,698,527 discloses ergostanone derivatives substituted with disaccharides as cholesterol absorption inhibitors, employed alone or in combination with certain other cholesterol lowering agents, which are useful in the treatment of hypercholesterolemia and related disorders.

WO 95/35277 describes substituted azetidinone hypocholesterolemic agents in combination with a cholesterol biosynthesis inhibitor.

EP-A-1 036 563 discloses a delayed-release oral formulation of dihydroxy open acid simvastatin which may optionally be combined with a broad range of additional active agents.

WO 01/96347, WO 02/50068, WO 02/50060 and WO 02/064094 belong to the prior art under Article 54 (3) EPC. WO 01/96347 discloses specific HMG CoA reductase inhibitors in combination with one or more other therapeutically useful and active agents. WO 02/50068 and WO 02/50060 describe diphenyl azetidinone derivatives as hypolipidemic agents, optionally in combination with one or more further active agents. WO 02/064094 is directed to a class of 2-aryloxy-2-arylalkanoic acids as PPAR alpha and/or gamma agonists in optional combination with one or more other active compounds, including HMG CoA reductase inhibitors.

Vascular conditions are often associated with thrombotic events sometimes resulting in myocardial infarction, stroke and ischemic attack. A thrombotic event is one associated with the formation or presence of a thrombus e.g. (blood clot), which results from an aggregation of blood factors, primarily platelets and fibrin with entrapment of cellular elements, frequently causing vascular obstruction at the point of its formation.

Blood coagulation is a process consisting of a complex interaction of various blood components, or factors, which eventually gives rise to a fibrin clot. Generally, the blood components which participate in what has been referred to as the coagulation "cascade" are proenzymes or zymogens, enzymatically inactive proteins, which are converted to proteolytic enzymes by the action of an activator, itself an activated clotting factor. Coagulation factors that have undergone such a conversion are generally referred to as "active factors", and are designated by the addition of the letter "a" to the name of the coagulation factor (e.g. fVIIa).

Activated factor X (fXa) is required to convert prothrombin to thrombin, which then converts fibrinogen to fibrin as a final stage in forming a fibrin clot. There are two systems or pathways that promote the activation of factor X. The "intrinsic pathway" refers to those reactions that lead to thrombin formation through utilization of factors present only in plasma. A series of protease-mediated activations ultimately generates factor IXa, which, in conjunction with factor VIIIa, cleaves factor X into Xa. An identical proteolysis is effected by fVIIa and its cofactor TF in the "extrinsic pathway" of blood coagulation. TF is a membrane bound protein and does not normally circulate in plasma. Upon vessel disruption, however, it is exposed and forms a complex with fVIIa to catalyze factor X activation or factor IX activation in the presence of $Ca^{2+}$ and phospholipid. While the relative importance of the two coagulation pathways in hemostasis is unclear, in recent years fVIIa and TF have been found to play a pivotal role in the initiation and regulation of blood coagulation.

FVII is a trace plasma glycoprotein that circulates in blood as a single-chain zymogen. The zymogen is catalytically inactive. Single-chain fVII may be converted to two-chain fVIIa by factor Xa, factor XIIa, factor IXa, fVIIa or thrombin *in vitro.* Factor Xa is believed to be the major physiological activator of fVII. Like several other plasma proteins involved in hemostasis, fVII is dependent on vitamin K for its activity which is required for the gamma-carboxylation of multiple glutamic acid residues that are clustered in the amino terminus of the protein. These gamma-carboxylated glutamic acids are required for the metal-associated interactions of fVII with phospholipids.

The conversion of zymogen fVII into the activated two-chain molecule occurs by cleavage of an internal Arg 152-Ile 153 peptide bond. In the presence of TF, phospholipids and calcium ions, the two-chain fVIIa rapidly activates factor X or factor IX by limited proteolysis.

It is often desirable to selectively block or inhibit the coagulation cascade in a patient. Blood modifiers such as heparin, coumarin, derivatives of coumarin, indandione derivatives, thrombin inhibitors, factor Xa inhibitors, modified fVII or other agents may be used.

The use of drugs to modify blood is beneficial in a number of vascular disease states including atherosclerosis. Proliferation of smooth muscle cells (SMCs) in the vessel wall is an important event in the formation of vascular lesions in atherosclerosis, after vascular reconstruction or in response to other vascular injury. SMC proliferation typically occurs within the first few weeks and up to six months after injury.

In about 30% or more of patients treated by angioplasty or bypass grafts, thrombosis and or SMC proliferation causes re-occlusion of the vessel. This closure of the vessel subsequent to surgery is known as restenosis.

Modified FVIIa has been shown to effectively suppress the restenosis process possibly as a result of a decreased procoagulant activity and thrombin generation initially after treatment of the constricted vessel (see e.g. US Patent No. 5,639,739).

Other blood modifiers have been used to treat thrombotic events associated with vascular conditions (see for example, WO 01/21/21259). Despite recent improvements in the treatment of vascular disease, there remains a need in the art for improved compositions and treatments.

SUMMARY OF THE INVENTION

In one embodiment of the present invention provides a composition comprising (a) at least one sterol absorption which is ezetimibe; and (b) at least one blood modifier for vascular conditions different from the component (a) above, namely a platelet inhibitor which is aspirin.

Pharmaceutical compositions for the treatment or prevention of a therosclerosis, hyperlipidaemia, vascular inflammation, hypertension, angina, cardiac arrythmias, stroke, diabetes, obesity of a mammal, comprising a therapeutically effective amount of the above compositions and a pharmaceutically acceptable carrier also are provided.

DETAILED DESCRIPTION

In one embodiment, the present invention is directed to compositions and pharmaceutical compositions as discussed in detail below.

The compositions and therapeutic combinations of the present invention can be administered to a mammal in need of such treatment in a therapeutically effective amount to treat "vascular conditions", namely atherosclerosis, hyperlipidaemia (including hypercholesterolaemia, hypertriglyceridaemia, sitosterolemia), vascular inflammation, hypertension, angina, cardiac arrhythmias, stroke, as well as conditions such diabetes, and for obesity. The compositions can be administered by any suitable means which produce contact of these compounds with the site of action in the body, for example in the plasma, liver or small intestine of a mammal or human.

"Blood modifiers" as used herein refers to aspirin, capable of altering the number of platelets per a given volume of blood, inhibiting platelet function, including but not limited to platelet adhesion, aggregation or factor release, or reducing platelet count in patients with abnormally high levels in certain hematological malignancies to levels approximating normal levels capable of impacting negatively upon the formation of blood clots, and decreasing blood viscosity.

Generally, a total dosage of the above-described aspirin can range from 1 to 3,000 mg/day, desirably from 1 to 1,000 mg/day and more desirably from 1 to 200 mg/day in single or 2-4 divided doses.

The compositions can be administered in a therapeutically effective amount of the blood modifier aspirin to treat the specified condition, for example in a daily dose preferably ranging from 1 to 1000 mg per day, and more preferably 5 to 200 mg per day, given in a single dose or 2-4 divided doses. The exact dose, however, is determined by the attending clinician and is dependent on such factors as the potency of the compound administered, the age, weight, condition and response of the patient.

The term "therapeutically effective amount" means that amount of a therapeutic agent of the composition, such as the blood modifier aspirin the sterol absorption inhibitor ezetimibe and other pharmacological or therapeutic agents described below, that will elicit a biological or medical response of a tissue, system, animal or mammal that is being sought by the administrator (such as a researcher, doctor or veterinarian) which includes alleviation of the symptoms of the condition or disease being treated and the prevention, slowing or halting of progression of the condition (for example a vascular condition as discussed above).

As used herein, "combination therapy" or "therapeutic combination" means the administration of two or more therapeutic agents, such as the blood modifier aspirin and the sterol absorption inhibitor ezetimibe, to prevent or treat vascular conditions. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating vascular and other conditions as discussed above. A potential advantage of the combination therapy disclosed herein may be a reduction in the required amount of an individual therapeutic compound or the overall total amount of therapeutic compounds that are effective in treating blood modifiers. By using a combination of therapeutic agents, the side effects of the individual compounds can be reduced as compared to a monotherapy, which can improve patient compliance. Also, therapeutic agents can be selected to provide a broader range of complimentary effects or complimentary modes of action.

As discussed above, the compositions, pharmaceutical compositions and therapeutic combinations of the present invention comprise a sterol absorption inhibitory, which is ezetimibe. As used herein, "sterol absorption inhibitor" means a compound capable of inhibiting the absorption of one or more sterols, including cholesterol, phytosterols (such as sitosterol, campesterol, stigmasterol and avenosterol), 5$\alpha$-stanols (such as cholestanol, 5$\alpha$-campestanol, 5$\alpha$-sitostanol), and mixtures thereof, when administered in a therapeutically effective (sterol absorption inhibiting) amount to a mammal or human.

The daily dose of the sterol absorption inhibitor ezetimibe can range from 0.1 to 1000 mg per day, preferably 0.25 to 50 mg/day, and more preferably about 10 mg per day, given in a single dose or 2-4 divided doses. The exact dose, however, is determined by the attending clinician and is dependent on the potency of the compound administered, the age, weight, condition and response of the patient.

For administration of pharmaceutically acceptable salts of the above compounds, the weights indicated above refer to the weight of the acid equivalent or the base equivalent of the therapeutic compound derived from the salt.

In one embodiment of the present invention, the compositions or therapeutic combinations can further comprise one or more pharmacological or therapeutic agents or drugs such as cholesterol biosynthesis inhibitors and/or lipid-lowering agents discussed below.

In another embodiment, the composition or treatment can further comprise one or more cholesterol biosynthesis inhibitors coadministered with or in combination with the blood modifier aspirin and sterol absorption inhibitor ezetimibe discussed above.

Examples of cholesterol biosynthesis inhibitors for use in the compositions, therapeutic combinations and methods of the present invention include competitive inhibitors of HMG CoA reductase, the rate-limiting step in cholesterol biosynthesis, squalene synthase inhibitors, squalene epoxidase inhibitors and mixtures thereof. Examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, rosuvastatin, rivastatin (sodium 7-(4-fluorophenyl)-2,6-diisopropyl-5-methoxymethylpyridin-3-yl)-3,5-dihydroxy-6-heptanoate CI-981 and pitavastatin (such as NK-104 of Negma Kowa of Japan); HMG CoA synthetase inhibitors, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin and simvastatin. The most preferred HMG CoA reductase inhibitor is simvastatin.

Generally, a total daily dosage of cholesterol biosynthesis inhibitor(s) can range from 0.1 to 160 mg per day, and preferably 0.2 to 80 mg/day in single or 2-3 divided doses.

In another preferred embodiment, the composition comprises the compound ezetimibe in combination with the blood modifier aspirin and one or more cholesterol biosynthesis inhibitors. Preferably the cholesterol biosynthesis inhibitor comprises one or more HMG CoA reductase inhibitors, such as, for example, lovastatin, pravastatin and/or simvastatin.

In another alternative embodiment, the compositions of the present invention can further comprise nicotinic acid (niacin) and/or derivatives thereof coadministered with or in combination with the blood modifier aspirin and the sterol absorption inhibitor ezetimibe discussed above.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from 500 to 10,000 mg/day, preferably 1000 to 8000 mg/day, and more preferably 3000 to 6000 mg/day in single or divided doses.

In another alternative embodiment, the compositions of the present invention can further comprise one or more AcylCoA:Cholesterol O-acyltransferase ("ACAT") Inhibitors, which can reduce LDL and HDL levels, coadminist re with or in combination with the blood modifier aspirin and the sterol absorption inhibitor ezetimibe discussed above. ACAT is an enzyme responsible for esterifying excess intracellular cholesterol and may reduce the synthesis of VLDL, which is a product of cholesterol esterification, and overproduction of apo B-100-containing lipoproteins.

Examples of useful ACAT inhibitors include avasimibe ([[2,4,6-tris(1-methylethyl)phenyl]acetyl]sulfamic acid, 2,6-bis(1-methylethyl)phenyl ester, formerly known as CI-1011), HL-004, lecimibide (DuP-128) and CL-277082 (N-(2,4-difluorophenyl)-N-[[4-(2,2-dimethylpropyl)phenyl]methyl]-N-heptylurea). See P. Chang et al., "Current, New and Future Treatments in Dyslipidaemia and Atherosclerosis", Drugs 2000 Jul;60(1); 55-93.

In another alternative embodiment, the compositions of the present invention can further comprise probucol or derivatives thereof (such as AGI-1067 and other derivatives disclosed in U.S. Patents Nos. 6,121,319 and 6,147,250), which can reduce LDL and HDL levels, coadministered with or in combination with the blood modifier aspirin and the sterol absorption inhibitor ezetimibe discussed above.

Generally, a total daily dosage of probucol or derivatives thereof can range from 10 to 2000 mg/day, and preferably 500 to 1500 mg/day in single or 2-4 divided doses.

In another alternative embodiment, the compositions of the present invention can further comprise low-density lipoprotein (LDL) receptor activators, coadministered with or in combination with the blood modifier aspirin and the sterol absorption inhibitor ezetimibe discussed above. Examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from 1 to 1000 mg/day in single or 2-4 divided doses.

In another alternative embodiment, the compositions of the present invention can further comprise fish oil, which contains Omega 3 fatty acids (3-PUFA), which can reduce VLDL and triglyceride levels, coadministered with or in combination with the blood modifier aspirin and the sterol absorption inhibitor ezetimibe discussed above. Generally, a total daily dosage of fish oil or Omega 3 fatty acids can range from 1 to 30 grams per day in single or 2-4 divided doses.

In another alternative embodiment, the compositions of the present invention can further comprise natural water soluble fibers, such as psyllium, guar, oat and pectin, which can reduce cholesterol levels, coadministered with or in combination with the blood modifier aspirin and the sterol absorption inhibitor ezetimibe discussed above. Generally, a total daily dosage of natural water soluble fibers can range from 0.1 to 10 grams per day in single or 2-4 divided doses.

In another alternative embodiment, the compositions of the present invention can further comprise plant sterols, plant stanols and/or fatty acid esters of plant stanols, such as sitostanol ester used in BENECOL® margarine, which can reduce cholesterol levels, coadministered with or in combination with the blood modifier aspirin and the sterol absorption inhibitor ezetimibe discussed above. Generally, a total daily dosage of plant sterols, plant stanols and/or fatty acid esters of plant stanols can range from 0.5 to 20 grams per day in single or 2-4 divided doses.

In another alternative embodiment, the compositions of the present invention can further comprise antioxidants, such as probucol, tocopherol, ascorbic acid, β-carotene and selenium, or vitamins such as vitamin $B_6$ or vitamin $B_{12}$, coadministered with or in combination with the blood modifier aspirin and the sterol absorption inhibitor ezetimibe discussed above. Generally, a total daily dosage of antioxidants or vitamins can range from 0.05 to 10 grams per day in single or 2-4 divided doses.

In another alternative embodiment, the compositions, therapeutic combinations of the present invention can further comprise one or more bile acid sequestrants (insoluble anion exchange resins), coadministered with or in combination with the blood modifier aspirin and the sterol absorption inhibitor ezetimibe discussed above.

Bile acid sequestrants bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of apo B/E (LDL) receptors which bind LDL from plasma to further reduce cholesterol levels in the blood.

Examples of suitable bile acid sequestrants include cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quatemized polystyrenes, saponins and mixtures thereof. Other useful bile acid sequestrants are disclosed in PCT Patent Applications Nos. WO 97/11345 and WO 98/57652, and U.S. Patents Nos. 3,692,895 and 5,703,188. Suitable inorganic cholesterol sequestrants include bismuth salicylate plus montmorillonite clay, aluminum hydroxide and calcium carbonate antacids.

Generally, a total daily dosage of bile acid sequestrant(s) can range from 1 to 50 grams per day, and preferably 2 to 16 grams per day in single or 2-4 divided doses.

Also useful with the present invention are compositions or therapeutic combinations that can further comprise at least one (one or more) activators for peroxisome proliferator-activated receptors (PPAR). The activators act as agonists for the peroxisome proliferator-activated receptors. Three subtypes of PPAR have been identified, and these are designated as peroxisome proliferator-activated receptor alpha (PPARα), peroxisome proliferator-activated receptor gamma (PPARγ) and peroxisome proliferator-activated receptor delta (PPARδ). It should be noted that PPARδ is also referred to in the literature as PPARβ and as NUC1, and each of these names refers to the same receptor.

PPARα regulates the metabolism of lipids. PPARα is activated by fibrates and a number of medium and long-chain fatty acids, and it is involved in stimulating β-oxidation of fatty acids. The PPARγ receptor subtypes are involved in activating the program of adipocyte differentiation and are not involved in stimulating peroxisome proliferation in the liver. PPARδ has been identified as being useful in increasing high density lipoprotein (HDL) levels in humans. See, e.g., WO 97/28149.

PPARα activator compounds are useful for, among other things, lowering triglycerides, moderately lowering LDL levels and increasing HDL levels. Useful examples of PPARα activators include fibrates.

Examples of suitable fibric acid derivatives ("fibrates") include clofibrate (such as ethyl 2-(p-chlorophenoxy)-2-methyl-propionate, for example ATROMID-S® Capsules which are commercially available from Wyeth-Ayerst); gemfibrozil (such as 5-(2,5-dimethylphenoxy)-2,2-dimethylpentanoic acid, for example LOPID® tablets which are commercially available from Parke Davis); ciprofibrate (C.A.S. Registry No. 52214-84-3, see U.S. Patent No. 3,948,973); bezafibrate (C.A.S. Registry No. 41859-67-0, see U.S. Patent No. 3,781,328); clinofibrate (C.A.S. Registry No. 30299-08-2, see U.S. Patent No. 3,716,583); binifibrate (C.A.S. Registry No. 69047-39-8, see BE 884722); lifibrol (C.A.S. Registry No. 96609-16-4); fenofibrate (such as TRICOR® micronized fenofibrate (2-[4-(4-chlorobenzoyl) phenoxy]-2-methyl-propanoic acid, 1-methylethyl ester) which is commercially available from Abbott Laboratories or LIPANTHYL® micronized fenofibrate which is commercially available from Labortoire Founier, France) and mixtures thereof. These compounds can be used in a variety of forms, including acid form, salt form, racemates, enantiomers, zwitterions and tautomers.

Other examples of PPARα activators useful with the practice of the present invention include suitable fluorophenyl compounds as disclosed in U.S. No. 6,028,109; certain substituted phenylpropionic compounds as disclosed in WO 00/75103 and PPARα activator compounds as disclosed in WO 98/43081.

Examples of suitable PPARγ activators include derivatives of glitazones or thiazolidinediones, such as, troglitazone (such as REZULIN® troglitazone (-5-[[4-[3,4-dihydro-6-hydroxy-2,5,7,8-tetramethyl-2H-1-benzopyran-2-yl)methoxy]phenyl] methyl]-2,4-thiazotidinedione) commercially available from Parke-Davis); rosiglitazone (such as AVANDIA® rosiglitazone maleate (-5-[[4-[2-(methyl-2-pyridinylamino)ethoxy] phenyl] methyl]-2,4-thiazolidinedione, (Z) -2-butenedioate) (1:1) commercially available from SmithKline Beecham) and pioglitazone (such as ACTOS™ pioglitazone hydrochloride (5-[[4-[2-(5-ethyl-2-pyridinyl)ethoxy]phenyl]methyl]-2,4-] thiazolidinedione monohydrochloride) commercially available from Takeda Pharmaceuticals). Other useful thiazolidinediones include ciglitazone, englitazone, darglitazone and BRL 49653 as disclosed in WO 98/05331; PPARγ activator compounds disclosed in WO 00/76488; and PPARγ activator compounds disclosed in U.S. Patent No. 5,994,554.

Other useful PPARγ activator compounds include certain acetylphenols as disclosed in U.S. Patent No. 5,859,051, certain quinoline phenyl compounds as disclosed in WO 99/20275 aryl compounds as disclosed by WO 99/38845; certain 1,4-disubstituted phenyl compounds as disclosed in WO 00/63161; certain aryl compounds as disclosed in WO 01/00579; benzoic acid compounds as disclosed in WO 01/12612 and WO 01/12187; and substituted 4-hydroxy-phenylalconic acid compounds as disclosed in WO 97/31907.

PPARδ compounds are useful for, among other things, lowering triglyceride levels or raising HDL levels. Examples of PPARδ activators include suitable thiazole and oxazole derivates, such as C.A.S. Registry No. 317318-32-4, as disclosed in WO 01/00603); certain fluoro, chloro or thio phenoxy phenylacetic acids as disclosed in WO 97/28149; suitable non-β-oxidizable fatty acid analogues as disclosed in U.S. Patent No. 5,093,365; and PPARδ compounds as disclosed in WO 99/04815.

Moreover, compounds that have multiple functionality for activating various combinations of PPARα, PPARγ and PPARδ are also useful with the practice of the present invention. Example include certain substituted aryl compounds as disclosed in U.S. Patent No. 6.248,781; WO 00/23416; WO 00/23415; WO 00/23425; WO 00/23445; WO 00/23451, and WO 00/63153, are described as being useful PPARα and/or PPARγ activator compounds. Other non-limiting examples of useful PPARα and/or PPARγ activator compounds include activator compounds as disclosed in WO 97/25042; activator compounds as disclosed in WO 00/63190; activator compounds as disclosed in WO 01/21181; biaryl-oxa(thia) zole compounds as disclosed in WO 01/16120; compounds as disclosed in WO 00/63196 and WO 00/63209; substituted 5-aryl-2,4-thiazolidinediones compounds as disclosed in U.S. Patent No. 6,008,237 arylthiazolidinedione and aryloxazolidinedione compounds as disclosed in WO 00/78312 and WO 00/78313G; GW2331 or (2-(4-[difluorophenyl]-1heptylureido)ethyl]phenoxy)-2-methylbutyric compounds as disclosed in WO 98/05331; aryl compounds as disclosed in U.S. Patent No. 6,166,049 oxazote compounds as disclosed in WO 01/17994; and dithiolane compounds as disclosed in WO 01/25225 and WO 01/25226.

Other useful PPAR activator compounds include substituted benzylthiazolidine-2,4-dione compounds as disclosed in WO 01/14349, WO 01/14350 and WO/01/04351; mercaptocarboxylic compounds as disclosed in WO 00/50392; ascofuranone compounds as disclosed in WO 00/53563; carboxylic compounds as disclosed in WO 99/46232; compounds as disclosed in WO 99/12534; benzene compounds as disclosed in WO 99/15520; o-anisamide compounds as disclosed in WO 01/21578; and PPAR activator compounds as disclosed in WO 01/40192.

The peroxisome proliferator-activated receptor(s) activator(s) are administered in a therapeutically effective amount to treat the specified condition, for example in a daily dose preferably ranging from 50 to 3000 mg per day, and more preferably 50 to 2000 mg per day, given in a single dose or 2-4 divided doses. The exact dose, however, is determined by the attending clinician and is dependent on such factors as the potency of the compound administered, the age, weight, condition and response of the patient.

In an alternative embodiment, the compositions of the present invention can further comprise one or more ileal bile acid transport ("IBAT") inhibitors (or apical sodium co-dependent bile acid transport ("ASBT") inhibitors) coadministered with or in combination with the blood modifier aspirin and the sterol absorption inhibitor ezetimibe discussed above. The IBAT inhibitors can inhibit bile acid transport to reduce LDL cholesterol levels. Suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727.

Generally, a total daily dosage of IBAT inhibitor(s) can range from 0.01 to 1000 mg/day, and preferably 0.1 to 50 mg/day in single or 2-4 divided doses.

In another alternative embodiment, the compositions of the present invention can further comprise one or more Cholesteryl Ester Transfer Protein ("CETP") Inhibitors coadministered with or in combination with the blood modifier aspirin and the sterol absorption inhibitor ezetimibe discussed above. CETP is responsible for the exchange or transfer of cholesteryl ester carrying HDL and triglycerides in VLDL.

Examples of suitable CETP inhibitors are disclosed in PCT Patent Application No. WO 00/38721 and U.S. Patent No. 6,147,090. Pancreatic cholesteryl ester hydrolase (pCEH) inhibitors such as WAY-121898 also can be coadministered with or in combination with the peroxiome proliferator-activated receptor activator and sterol absorption inhibitor ezetimibe discussed above.

Generally, a total daily dosage of CETP inhibitor(s) can range from 0.01 to 1000 mg/day, and preferably 0.5 to 20 mg/kg body weight/day in single or divided doses.

Also useful with the present invention are compositions or therapeutic combinations that further comprise hormone replacement agents and compositions. Useful hormone agents and compositions for hormone replacement therapy of the present invention include androgens, estrogens, progestins, their pharmaceutically acceptable salts and derivatives. Combinations of these agents and compositions are also useful.

The dosage of androgen and estrogen combinations vary, desirably from 1 mg to 4 mg androgen and from 1 mg to 3 mg estrogen. Examples include, androgen and estrogen combinations such as the combination of esterified estrogens (sodium estrone sulfate and sodium equilin sulfate) and methyltestosterone (17-hydroxy-17-methyl-, (17B)-androst-4-en-3-one) available from Solvay Pharmaceuticals, Inc., Marietta, GA, under the tradename Estratest.

Estrogens and estrogen combinations may vary in dosage from 0.01 mg up to 8 mg, desirably from 0.3 mg to 3.0 mg. Examples of useful estrogens and estrogen combinations include:

(a) the blend of nine (9) synthetic estrogenic substances including sodium estrone sulfate, sodium equilin sulfate, sodium 17 α-dihydroequilin sulfate, sodium 17 α-estradiol sulfate, sodium 17 β-dihydroequilin sulfate, sodium 17 α-dihydroequilenin sulfate, sodium 17 β-dihydroequilenin sulfate, sodium equilenin sulfate and sodium 17 β-estradiol sulfate; available from Duramed Pharmaceuticals, Inc., Cincinnati, OH, under the tradename Cenestin;

(b) ethinyl estradiol (19-nor-17α-pregna-1,3,5(10)-trien-20-yne-3,17-diol; available by Schering Plough Corporation, Kenilworth, NJ, under the tradename Estinyl;

(c) esterified estrogen combinations such as sodium estrone sulfate and sodium equilin sulfate; available from Solvay under the tradename Estratab and from Monarch Pharmaceuticals, Bristol, TN, under the tradename Menest™;

(d) estropipate (piperazine estra-1,3,5(10)-trien-17-one, 3-(sulfooxy)-estrone sulfate); available from Pharmacia & Upjohn, Peapack, NJ, under the tradename Ogen™ and from Women First Health Care, Inc., San Diego, CA, under the tradename Ortho-Est™; and

(e) conjugated estrogens (17 α-dihydroequilin, 17 α-estradiol, and 17 β-dihydroequilin); available from Wyeth-Ayerst Pharmaceuticals, Philadelphia, PA, under the tradename Premarin™.

Progestins and estrogens may also be administered with a variety of dosages, generally from about .05 to about 2.0 mg progestin and about .001 mg to about 2 mg estrogen, desirably from about .1 mg to about 1 mg progestin and about 0.01 mg to about .5 mg estrogen. Examples of progestin and estrogen combinations that may vary in dosage and regimen include:

(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename Activella™;

(b) the combination of levonorgestrel (d(-)-13 β-ethyl-17 α-ethinyl-17 β-hydroxygon- 4-en-3-one) and ethinyl estradial; available from Wyeth-Ayerst under the tradename Alesse™, from Watson Laboratories, Inc., Corona, CA, under the tradenames Levora™ and Trivora™, Monarch Pharmaceuticals, under the tradename Nordette™, and from Wyeth-Ayerst under the tradename Triphasil™;

(c) the combination of ethynodiol diacetate (19-nor-17 α-pregn-4-en-20-yne-3β, 17-diol diacetate) and ethinyl estradiol; available from G.D. Searle & Co., Chicago, IL, under the tradename Demulen™ and from Watson under the tradename Zovia™;

(d) the combination of desogestrel (13-ethyl-11- methylene-18,19-dinor-17 α-pregn- 4-en- 20-yn-17-ol) and ethinyl estradiol; available from Organon under the tradenames Desogen™ and Mircette™, and from Ortho-McNeil Pharmaceutical, Raritan, NJ, under the tradename Ortho-Cept™;

(e) the combination of norethindrone and ethinyl estradiol ; available from Parke-Davis, Morris Plains, NJ, under the tradenames Estrostep™ and femhrt, from Watson under the tradenames Microgestin™, Necon™, and Tri-Norinyl™, from Ortho-McNeil under the tradenames Modicon™ and Ortho-Novum™, and from Warner Chilcott Laboratories, Rockaway, NJ, under the tradename Ovcon™,

(f) the combination of norgestrel ((±)-13-ethyl-17-hydroxy-18, 19-dinor-17 α-preg-4-en-20-yn-3-one) and ethinyl estradiol; available from Wyeth-Ayerst under the tradenames Ovral™ and Lo/Ovral™, and from Watson under the tradenames Ogestrel™ and Low-Ogestrel™,

(g) the combination of norethindrone, ethinyl estradiol, and mestranol (3-methoxy-19-nor-17 α-pregna-1,3,5(10)-trien-20-yn-17-ol) ; available from Watson under the tradenames Brevicon™ and Nodnyl™;

(h) the combination of 17 β-estradiol (estra-1,3,5(10)-triene-3,17 β-diol) and micronized norgestimate (17 α-17-(Acetyloxyl)-13-ethyl-18,19-dinorpregn-4-en-20-yn-3-one3-oxime); available from Ortho-McNeil under the tradename Ortho-Pretest™;

(i) the combination of norgestimate (18,19-dinor-1 7-pregn-4-en-20-yn-3-one, 17-(acety)oxy)-13-ethy)-.oxime, (17 (α)-(+)-) and ethinyl estradiol; available from Ortho-McNeil under the tradenames Ortho Cyclen™ and Ortho Tri-Cyclen™; and

(j) the combination of conjugated estrogens (sodium estrone sulfate and sodium equilin sulfate) and medroxyprogesterone acetate (20-dione, 17-(acetyloxy)-6-methyl-, (6(α))-pregn-4-ene-3); available from Wyeth-Ayerst under the tradenames Premphase™ and Prempro™.

In general, a dosage of progestins may vary from about .05 mg to about 10 mg or up to about 200 mg if microsized progesterone is administered. Examples of progestins include norethindrone; available from ESI Lederle, Inc., Philadelphia, PA, under the tradename Aygestin™, from Ortho-McNeil under the tradename Micronor™, and from Watson under the tradename Nor-QD™; norgestrel; available from Wyeth-Ayerst under the tradename Ovrette™; micronized progesterone (pregn-4-ene-3, 20-dione); available from Solvay under the tradename Prometrium™; and medroxyprogesterone acetate; available from Pharmacia & Upjohn under the tradename Provera™.

The compositions, therapeutic combinations of the present invention can further comprise one or more obesity control medications. Useful obesity control medications include, drugs that reduce energy intake or suppress appetite, drugs that increase energy expenditure and nutrient-partitioning agents. Suitable obesity control medications include, noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); an alpha-blocking agent; a kainite or AMPA receptor antagonist; a leptin-lipolysis stimulated receptor; a phosphodiesterase enzyme inhibitor; a compound having nucleotide sequences of the mahogany gene; a fibroblast growth factor-10 polypeptide; a monoamine oxidase inhibitor (such as befloxatone, moclobemide, brofaromine, phenoxathine, esuprone, befol, toloxatone, pirlindol, amiflamine, sercloremine, bazinaprine, lazabemide, milacemide and caroxazone); a compound for increasing lipid metabolism (such as evodiamine compounds); and a lipase inhibitor (such as orlistat). Generally, a total dosage of the above-described obesity control medications can range from 1 to 3,000 mg/day, desirably from 1 to 1,000 mg/day and more desirably from 1 to 200 mg/day in single or 2-4 divided doses.

The compositions, therapeutic combinations of the present invention can further comprise one or more cardiovascular agents which are chemically or structurally different from the sterol absorption inhibitor ezetimibe and the blood modifier aspirin discussed above, for example, they contain one or more different atoms, have a different arrangement of atoms or a different number of one or more atoms than the sterol absorption inhibitory ezetimibe discussed below. Useful cardiovascular agents include to calcium channel blockers (clentiazem maleate, amiodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amiodipine maleate, bevantolol hydrochloride); angiotensin II receptor antagonists (candesartan, irbesartan, losartan potassium, candesartan cilexetil, telmisartan); anti-anginal agents (amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions, therapeutic combinations of the present invention can further comprise one or more antidiabetic medications for reducing blood glucose levels in a human. Useful antidiabetic medications include, drugs that reduce energy intake or suppress appetite, drugs that increase energy expenditure and nutrient-partitioning agents. Suitable antidiabetic medications include, sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), thiazolidinedione (such as troglitazone, rosiglitazone, pioglitazone, ciglitazone, englitazone, and darglitazone), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Compositions and therapeutic combinations of the present invention having the above-described sterol absorption inhibitor ezetimibe are also useful for treating or preventing vascular inflammation or for reducing c-reactive protein levels in a mammal in need of such treatment. Vascular inflammation refers to arterial damage and bodily responses thereto. For example, cholesteryl esters are a major component of atherosclerotic lesions which results in vascular inflammation and an increase in plasma c-reactive protein levels. The inhibition of cholesteryl ester formation and reduction of serum cholesterol can inhibit the progression of atherosclerotic lesion formation, thereby treating or preventing vascular inflammation. Moreover, these sterol absorption inhibitor ezetimibe is useful lowering or controlling c-reactive protein blood levels in a mammal to less than about 3.4 mg/dL, desirably to less than 1.0 mg/dL, and more desirably to less than 0.4 mg/dL.

Mixtures of any of the pharmacological or therapeutic agents described above can be used in the compositions and therapeutic combinations of these other embodiments of the present invention.

The compositions and therapeutic combinations of the present invention can be administered to a mammal in need of such treatment in a therapeutically effective amount to treat vascular conditions such as vascular conditions. The compositions and treatments can be administered by any suitable means that produce contact of these compounds with the site of action in the body, for example in the plasma, liver or small intestine of a mammal.

The daily dosage for the various compositions and therapeutic combinations described above can be administered to a patient in a single dose or in multiple subdoses, as desired. Subdoses can be administered 2 to 6 times per day, for example. Sustained release dosages can be used. Where the blood modifier aspirin and the sterol absorption inhibitor ezetimibe are administered in separate dosages, the number of doses of each component given per day may not necessarily be the same, e.g., one component may have a greater duration of activity and will therefore need to be administered less frequently.

The pharmaceutical treatment compositions and therapeutic combinations of the present invention can further comprise one or more pharmaceutically acceptable carriers, one or more excipients and/or one or more additives. Examples of pharmaceutically acceptable carriers include solids and/or liquids such as ethanol, glycerol, water. The amount of carrier in the treatment composition can range from 5 to 99 weight percent of the total weight of the treatment composition or therapeutic combination. Examples of suitable pharmaceutically acceptable excipients and additives include non-toxic compatible fillers, binders such as starch, disintegrants, buffers, preservatives, anti-oxidants, lubricants, flavorings, thickeners, coloring agents, emulsifiers. The amount of excipient or additive can range from 0.1 to 90 weight percent of the total weight of the treatment composition or therapeutic combination. One skilled in the art would understand that the amount of carrier(s), excipients and additives (if present) can vary.

The treatment compositions of the present invention can be administered in any conventional dosage form, preferably an oral dosage form such as a capsule, tablet, powder, cachet, suspension or solution. The formulations and pharmaceutical compositions can be prepared using conventional pharmaceutically acceptable and conventional techniques. Several examples of preparation of dosage formulations are provided below.

The following formulations exemplify some of the dosage forms of this invention. In each formulation, the term "Active Compound I" designates the sterol absorption the inhibitor described herein above and the term "Active Compound II" designates the blood modifier described herein above.

EXAMPLE

| | Tablets | |
|---|---|---|
| No. | Ingredient | mg/tablet |
| 1 | Active Compound I | 10 |
| 2 | Lactose monohydrate NF | 55 |
| 3 | Microcrystalline cellulose NF | 20 |
| 4 | Povidone (K29-32) USP | 4 |
| 5 | Croscarmellose sodium NF | 8 |
| 6 | Sodium lauryl sulfate | 2 |
| 7 | Magnesium stearate NF | 1 |
| | Total | 100 |

In the present invention, the above-described tablet can be coadministered with a tablet, capsule, etc. comprising a dosage of Active Compound II, for example a blood modifier as described above.

Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

For coadministration in separate tablets or capsules, representative formulations comprising the sterol absorption inhibitor such as are discussed above are well known in the art and representative formulations comprising the blood modifier such as are discussed above are well known in the art. It is contemplated that where the two active ingredients are administered as a single composition, the dosage forms disclosed above for sterol absorption inhibitor may readily be modified using the knowledge of one skilled in the art.

Since the present invention relates to treating vascular conditions or reducing the plasma sterol (especially cholesterol) concentrations or levels or controlling properties of the blood, such as viscosity, by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising the blood modifier aspirin and a separate pharmaceutical composition comprising the sterol absorption inhibitor ezetimibe as described above. The kit will preferably include directions for the administration of the separate components. The kit form is particularly advantageous when the separate components must be administered in different dosage forms (e.g., oral and parenteral) or are administered at different dosage intervals.

The treatment compositions and therapeutic combinations of the present invention can inhibit the intestinal absorption of cholesterol in mammals, and can be useful in the treatment and/or prevention of vascular conditions, such as vascular inflammation, atherosclerosis, hypercholesterolemia and sitosterolemia, stroke, vascular conditions and lowering of plasma levels of cholesterol in mammals, in particular in humans.

In another embodiment of the present invention, the compositions and therapeutic combinations of the present invention can inhibit sterol absorption or reduce plasma concentration of at least one sterol selected from the group consisting of phytosterols (such as sitosterol, campesterol, stigmasterol and avenosterol), $5\alpha$-stanols (such as cholestanol, $5\alpha$-campestanol, $5\alpha$-sitostanol), cholesterol and mixtures thereof. The plasma concentration can be reduced by administering to a mammal in need of such treatment an effective amount of at least one treatment composition or therapeutic combination comprising at least one blood modifier and at least one sterol absorption inhibitor described above. The reduction in plasma concentration of sterols can range from 1 to 70 percent, and preferably 10 to 50 percent. Methods of measuring serum total blood cholesterol and total LDL cholesterol are well known to those skilled in the art and for example include those disclosed in PCT WO 99/38498 at page 11, (Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999).

Illustrating the invention is the following example. Unless otherwise indicated, all parts and percentages in the following examples, as well as throughout the specification, are by weight.

## EXAMPLE

## PREPARATION OF COMPOUND OF FORMULA (II)

Step 1): To a solution of (S)-4-phenyl-2-oxazolidinone (41 g, 0.25 mol) in $CH_2Cl_2$ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g, 0.3 mol) was added as a solution in $CH_2Cl_2$ (375 ml) dropwise over 1 h, and the reaction was allowed to warm to 22°C. After 17 h, water and $H_2SO_4$ (2N, 100 ml), was added the layers were separated, and the organic layer was washed sequentially with NaOH (10%), NaCl (sat'd) and water. The organic layer was dried over $MgSO_4$ and concentrated to obtain a semicrystalline product.

Step 2): To a solution of $TiCl_4$ (18.2 ml, 0.165 mol) in $CH_2Cl_2$ (600 ml) at 0°C, was added titanium isopropoxide (16.5 ml, 0.055 mol). After 15 min, the product of Step 1 (49.0 g, 0.17 mol) was added as a solution in $CH_2Cl_2$ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in $CH_2Cl_2$ dropwise over 15 min, the reaction mixture was allowed to warm to 0°C, and $H_2SO_4$ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis(trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, $CH_3OH$ (10 ml), was added. The reaction mixture was washed with HCl (1N), $NaHCO_3$ (1N) and NaCl (sat'd.), and the organic layer was dried over $MgSO_4$.

Step 4): To a solution of the product of Step 3 (0.94 g, 2.2 mmol) in $CH_3OH$ (3 ml), was added water (1 ml) and $LiOH \cdot H_2O$ (102 mg, 2.4 mmole). The reaction mixture was stirred at 22°C for 1 h and then additional $LiOH \cdot H_2O$ (54 mg, 1.3 mmole) was added. After a total of 2 h, HCl (1N) and EtOAc was added, the layers were separated, the organic layer was dried and concentrated in *vacuo.* To a solution of the resultant product (0.91 g, 2.2 mmol) in $CH_2Cl_2$ at 22°C, was added ClCOCOCl (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1 M in THF, 4.4 ml, 4.4 mmol) and $ZnCl_2$ (0.6 g, 4.4 mmol) at 4°C, was added tetrakis (triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:
HRMS calc'd for $C_{24}H_{19}F_2NO_3$ = 408.1429, found 408.1411.

Step 6): To the product of Step 5 (0.95 g, 1.91 mmol) in THF (3 ml), was added (R)-tetrahydro-1-methyl-3,3-diphenyl-1 H,3H-pyrrolo-[1,2-c][1,3,2] oxazaborole (120 mg, 0.43 mmol) and the mixture was cooled to -20°C. After 5 min, borohydride-dimethylsulfide complex (2M in THF, 0.85 ml, 1.7 mmol) was added dropwise over 0.5 h. After a total of 1.5 h , $CH_3OH$ was added followed by HCl (1N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. $^1$H in $CDCl_3$ d H3 = 4.68. J = 2.3 Hz. CI ($M^+H$) 500.

Use of (S)-tetra-hydro-1-methyl-3,3-diphenyl-1H,3H-pyrrolo-[1,2-c][1,3,2] oxazaborole gives the corresponding 3(R)-hydroxypropyl azetidinone (compound 6B-1). $^1$H in $CDCl_3$ d H3 = 4.69. J = 2.3 Hz. CI ($M^+H$) 500.

To a solution of compound 6A-1 (0.4 g, 0.8 mmol) in ethanol (2 ml), was added 10% Pd/C (0.03 g) and the reaction mixture was stirred under a pressure (60 psi) of $H_2$ gas for 16 h. The reaction mixture was filtered and the solvent was concentrated to obtain compound 6A. Mp 164-166°C; CI ($M^+H$) 410. $[\alpha]_D^{25}$ = -28.1° (c 3, $CH_3OH$). Elemental analysis calc'd for $C_{24}H_{21}F_2NO_3$: C 70.41; H 5.17; N 3.42; found C 70.25; H 5.19; N 3.54.

Similarly treat compound 6B-1 to obtain compound 6B. Mp 129.5-132.5°C; CI ($M^+H$) 410. Elemental analysis calc'd for $C_{24}H_{21}F_2NO_3$: C 70.41; H 5.17; N 3.42; found C 70.30; H 5.14; N 3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of $H_2$ gas for 16 h. The reaction mixture was filtered and the solvent was concentrated to afford a 1:1 mixture of compounds 6A and 6B.

**Claims**

**1.** A composition comprising:

(a) at least one sterol absorption inhibitor which is ezetimibe, and
(b) at least one blood modifier for vascular conditions which is different from component (a) above, namely a platelet inhibitor, wherein the platelet inhibitor is aspirin.

**2.** The composition of claim 1, further comprising a component selected from at least one cholesterol biosynthesis inhibitor, at least one bile acid sequestrant, at least one low-density lipoprotein receptor activator, at least one Omega 3 fatty acid, at least one natural water soluble fiber, and at least one antioxidant or vitamin.

**3.** The composition of claim 2, wherein the at least one cholesterol biosynthesis inhibitor comprises at least one HMG CoA reductase inhibitor.

**4.** The composition of claim 3, wherein the at least one HMG CoA reductase inhibitor is simvastatin.

**5.** Pharmaceutical composition comprising a therapeutically effective amount of the composition of claim 1 and a pharmaceutically acceptable carrier.

**6.** Use of a composition of claim 1 for the manufacture of a medicament effective in the treatment or prevention of atherosclerosis, hyperlipidaemia, vascular inflammation, hypertension, angina, cardiac arrythmias, stroke diabetes, obesity.

**7.** Pharmaceutical composition of claim 5 or use of claim 6, wherein in the pharmaceutical composition or medicament the amount of the at least one blood modifier is adapted for administration to a mammal in an amount of about 1-1000 mg/day.

8. Pharmaceutical composition of claim 5 or use of claim 6, wherein in the pharmaceutical composition or medicament the amount of the at least one sterol absorption inhibitor is adapted for administration to a mammal in an amount of about 0.1-1000 mg/day.

**Patentansprüche**

1. Zusammensetzung, umfassend:

   (a) mindestens einen Sterolabsorptionsinhibitor, wobei es sich um Ezetimib handelt, und
   (b) mindestens ein Blutmodifikationsmittel für vaskuläre Zustände, das sich von Komponente (a) oben unterscheidet, nämlich einen Thrombozyteninhibitor, wobei der Thrombozyteninhibitor Aspirin ist.

2. Zusammensetzung gemäß Anspruch 1, weiterhin umfassend eine Komponente ausgewählt aus mindestens einem Cholesterin-Biosyntheseinhibitor, mindestens einem Gallensäuresequestrationsmittel, mindestens einem Niedrigdichte-Lipoprotein-Rezeptoraktivator, mindestens einer Omega-3-Fettsäure, mindestens einer natürlichen wasserlöslichen Faser und mindestens einem Antioxidans oder Vitamin.

3. Zusammensetzung gemäß Anspruch 2, wobei der mindestens eine Cholesterin-Biosyntheseinhibitor mindestens einen HMG CoA-Reduktaseinhibitor umfaßt.

4. Zusammensetzung gemäß Anspruch 3, wobei der mindestens eine HMG CoA-Reduktaseinhibitor Simvastatin ist.

5. Pharmazeutische Zusammensetzung, umfassend eine therapeutisch effektive Menge der Zusammensetzung gemäß Anspruch 1 und einen pharmazeutisch annehmbaren Träger.

6. Verwendung einer Zusammensetzung gemäß Anspruch 1 für die Herstellung eines Medikaments, das für die Behandlung oder Prävention von Arteriosklerose, Hyperlipidämie, vaskulären Entzündungen, Bluthochdruck, Angina, Herzarrhythmien, Schlaganfall, Diabetes und Fettsucht effektiv ist.

7. Pharmazeutische Zusammensetzung gemäß Anspruch 5 oder Verwendung gemäß Anspruch 6, wobei in der pharmazeutischen Zusammensetzung oder dem Medikament die Menge des mindestens einen Blutmodifikationsmittels für eine Verabreichung an einen Säuger in einer Menge von ungefähr 1 bis 1.000 mg/Tag angepaßt ist.

8. Pharmazeutische Zusammensetzung gemäß Anspruch 5 oder Verwendung gemäß Anspruch 6, wobei in der pharmazeutischen Zusammensetzung oder dem Medikament die Menge des mindestens einen Sterolabsorptionsinhibitors für eine Verabreichung an einen Säuger in einer Menge von ungefähr 0,1 bis 1.000 mg/Tag angepaßt ist.

**Revendications**

1. Composition comprenant :

   a) au moins un inhibiteur de l'absorption des stérols, qui est de l'ézétimibe,
   b) et au moins un agent de modification du sang pour affections vasculaires, qui est différent du composant (a) mentionné ci-dessus, à savoir un inhibiteur plaquettaire, lequel inhibiteur plaquettaire est de l'aspirine.

2. Composition conforme à la revendication 1, comprenant en outre un composant choisi parmi au moins un inhibiteur de la biosynthèse du cholestérol, au moins un agent séquestrant les acides biliaires, au moins un activateur des récepteurs de lipoprotéines basse densité, au moins un acide gras oméga-3, au moins une sorte de fibres naturelles hydrosolubles, et au moins un anti-oxydant ou une vitamine.

3. Composition conforme à la revendication 2, dans laquelle ledit inhibiteur de la biosynthèse du cholestérol au nombre d'au moins un comprend au moins un inhibiteur de l'HMG-CoA réductase.

**4.** Composition conforme à la revendication 3, dans laquelle ledit inhibiteur de l'HMG-CoA réductase au nombre d'au moins un est de la sim-vastatine.

**5.** Composition pharmaceutique comprenant une composition conforme à la revendication 1, en une quantité à effet thérapeutique, et un véhicule pharmacologiquement admissible.

**6.** Utilisation d'une composition conforme à la revendication 1, en vue de la fabrication d'un médicament efficace dans le traitement ou la prévention de l'athérosclérose, de l'hyperlipidémie, d'une inflammation vasculaire, de l'hypertension, d'une angine, d'une arythmie cardiaque, d'une attaque, d'un diabète ou de l'obésité.

**7.** Composition pharmaceutique conforme à la revendication 5, ou utilisation conforme à la revendication 6, dans laquelle, dans la composition pharmaceutique ou dans le médicament, la quantité dudit agent de modification du sang au nombre d'au moins un est ajustée pour qu'on puisse en administrer à un mammifère une quantité d'à peu près 1 à 1000 mg/jour.

**8.** Composition pharmaceutique conforme à la revendication 5, ou utilisation conforme à la revendication 6, dans laquelle, dans la composition pharmaceutique ou dans le médicament, la quantité dudit inhibiteur de l'absorption de stérols au nombre d'au moins un est ajustée pour qu'on puisse en administrer à un mammifère une quantité d'à peu près 0,1 à 1000 mg/jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5767115 A
- US 5624920 A
- US 5668990 A
- US 5656624 A
- US 5688787 A
- US 5846966 A
- US 5661145 A
- WO 0038725 A
- US 5698527 A
- WO 9535277 A
- EP 1036563 A
- WO 0196347 A
- WO 0250068 A
- WO 0250060 A
- WO 02064094 A
- US 5639739 A
- WO 012121259 A
- US 6121319 A
- US 6147250 A
- WO 9711345 A
- WO 9857652 A
- US 3692895 A
- US 5703188 A
- WO 9728149 A
- US 3948973 A
- US 3781328 A
- US 3716583 A
- BE 884722
- US 6028109 A
- WO 0075103 A
- WO 9843081 A
- WO 9805331 A
- WO 0076488 A
- US 5994554 A
- US 5859051 A
- WO 9920275 A
- WO 9938845 A
- WO 0063161 A
- WO 0100579 A
- WO 0112612 A

- WO 0112187 A
- WO 9731907 A
- WO 0100603 A
- US 5093365 A
- WO 9904815 A
- US 6248781 B
- WO 0023416 A
- WO 0023415 A
- WO 0023425 A
- WO 0023445 A
- WO 0023451 A
- WO 0063153 A
- WO 9725042 A
- WO 0063190 A
- WO 0121181 A
- WO 0116120 A
- WO 0063196 A
- WO 0063209 A
- US 6008237 A
- WO 0078312 A
- WO 0078313G A
- US 6166049 A
- WO 0117994 A
- WO 0125225 A
- WO 0125226 A
- WO 0114349 A
- WO 0114350 A
- WO 0104351 A
- WO 0050392 A
- WO 0053563 A
- WO 9946232 A
- WO 9912534 A
- WO 9915520 A
- WO 0121578 A
- WO 0140192 A
- WO 0038727 A
- WO 0038721 A
- US 6147090 A
- WO 9938498 A

**Non-patent literature cited in the description**

- **P. CHANG et al.** Current, New and Future Treatments in Dyslipidaemia and Atherosclerosis. *Drugs,* July 2000, vol. 60 (1), 55-93

- **M. HUETTINGER et al.** Hypolipidemic activity of HOE-402 is Mediated by Stimulation of the LDL Receptor Pathway. *Arterioscler. Thromb.,* 1993, vol. 13, 1005-12

- **H. GYLLING et al.** Serum Sterols During Stanol Ester Feeding in a Mildly Hypercholesterolemic Population. *J. Lipid Res.,* 1999, vol. 40, 593-600